# EUROPEAN PATENT APPLICATION

(11) **EP 3 903 771 A1**
(43) Date of publication of application: **03.11.2021**
(21) Application number: 19904241.7
(22) Date of filing: 27.12.2019
(51) Int. Cl.: A61K 9/50, A61K 8/11, A61K 9/70, A61K 31/593, B01J 13/02, D06M 23/12

(54) **MATERIAL THAT INCORPORATES VITAMIN D FOR THE SUBSEQUENT RELEASE THEREOF AND METHOD FOR OBTAINING THE MATERIAL**

(30) Priority: 27.12.2018 CL 20183823
(71) Applicant: Universidad de Santiago de Chile, Santiago (CL); Comercializadora Textil la Joya Design Limitada, Lo Barnechea, Santiago (CL)
(72) Inventor: BUSTOS CERDA, Rubén Osvaldo, Estación Central Santiago (CL)
(74) Representative: Herrero & Asociados, S.L.
(86) International application number: PCT/CL2019/050156
(87) International publication number: WO 2020/132773

(57) **Abstract**

The present invention relates to the field of materials, more specifically to the treatment of materials for the release of active compounds, and in particular provides a material for the release of vitamin D to the skin, and a method for obtaining said material.

## Description

### TECHNICAL FIELD OF THE INVENTION

The present invention relates to the field of materials, more specifically to the treatment of materials for incorporation of active ingredients thereto, and in particular provides a material that includes vitamin D microparticles, intended to be released from this material directly to and incorporated into the skin, as well as the method for obtaining said material.

### BACKGROUND OF THE INVENTION

In the field of the materials industry, materials that allow the administration of active compounds through the skin have recently gained relevance. In particular, vitamin D has been shown to be a relevant agent for preventing the occurrence of diseases such as osteopenia or osteoporosis.

However, in the case of workers who carry out their work exposed to sunlight, current regulations require the application of sunscreen, which inhibits the synthesis of vitamin D at the skin level.

Materials that allow the release of active compounds directly to the skin have been described in the state of the art. For example, document US 7,956,025 describes a method for obtaining a fabric that allows the release of active compounds for cosmetic purposes to the skin. Said document only describes the manufacture of fabrics containing microcapsules comprising chitosan as matrix material and various active ingredients, among which vitamin D is not mentioned.

Consequently, a material that allows the release of vitamin D upon contact with the skin is required, as well as a method for its manufacture, in order to overcome the previously identified deficiency of the technique.

### SUMMARY OF THE INVENTION

The present invention provides a material for the release of vitamin D through the skin characterized in that it comprises: a fabric; and microcapsules containing vitamin D dispersed in said fabric and fixed thereto, wherein each of said microcapsules is formed by an amphipathic polymeric matrix encapsulating said vitamin D.

In a preferred embodiment, the material is characterized in that said fabric is selected from the group consisting of cotton fabric, polyester fiber fabric, as well as combinations thereof.

In another preferred embodiment, the material is characterized in that said fabric comprises copper microfibers.

In a further preferred embodiment, the material is characterized in that said polymeric matrix comprises an amphipathic polymer that is selected from the group consisting of sodium caseinate, acacia gum, and Capsul®, as well as combinations thereof. In a more preferred embodiment, the material is characterized in that said amphipathic polymer is sodium caseinate. In another more preferred embodiment, the material is characterized in that said polymeric matrix comprises an emulsifying agent with a hydrophilic-lipophilic balance between 10 and 20. In an even more preferred embodiment, the material is characterized in that said emulsifying agent is polysorbate 20.

In another preferred embodiment, the material is characterized in that said microcapsules have a diameter size between 0.7 microns and 3.2 microns.

In a preferred embodiment, the material is characterized in that the concentration of said microcapsules in said fabric is between 0.5 and 2 mg of vitamin D per square meter of fabric.

The present invention further provides a method for obtaining a material for the release of vitamin D through the skin, characterized in that it comprises the steps of: obtaining microcapsules containing vitamin D encapsulated in an amphipathic polymeric matrix; and fixing said microcapsules containing vitamin D to a fabric.

In a preferred embodiment, the method is characterized in that the step of obtaining said microcapsules containing vitamin D encapsulated in an amphipathic polymeric matrix comprises the steps of: obtaining an oil phase containing vitamin D; obtaining an aqueous phase containing an aqueous solution of a precursor of said amphipathic polymeric matrix; adding said oil phase to said aqueous phase while homogenizing the mixture; and recovering said microcapsules containing vitamin D.

In a more preferred embodiment, the method is characterized in that the volume ratio between said oil phase and said aqueous phase is between 1:10 and 1:2.

In another more preferred embodiment, the method is characterized in that said oil phase comprises an oil solution of vitamin D. In an even more preferred embodiment, the method is characterized in that said oil solution of vitamin D comprises a solvent that is selected from the group consisting of liquid petroleum jelly, vegetable oil, triolein, as well as combinations thereof.

In an additional more preferred embodiment, the method is characterized in that said oil phase contains a concentration of vitamin D between 100 ppm and 1000 ppm.

In another more preferred embodiment, the method is characterized in that said precursor of said polymeric matrix comprises an amphipathic polymer that is selected from the group consisting of sodium caseinate, acacia gum, and Capsul®, as well as combinations thereof. In an even more preferred embodiment, the method is characterized in that said amphipathic polymer is sodium caseinate. In an even more preferred embodiment, the method is characterized in that the concentration of sodium caseinate in said aqueous phase is between 7% and 15% w/w.

In a more preferred embodiment, the method is characterized in that said aqueous phase additionally contains an emulsifying agent having a hydrophilic-lipophilic balance between 10 and 20. In an even more preferred embodiment, the method is characterized in that said emulsifying agent is polysorbate 20. In an even more preferred embodiment, the method is characterized in that the concentration of polysorbate 20 in said aqueous phase is between 7% and 15% w/w.

In another preferred embodiment of the invention, the method is characterized in that the step of obtaining said aqueous phase comprises homogenizing the precursor of said polymeric matrix and water by means of a dispersing homogenizing apparatus. In an even more preferred embodiment, the method is characterized in that said homogenization is carried out at a temperature between 60°C and 80°C.

In a more preferred embodiment, the method is characterized in that said homogenization is carried out by means of a dispersing homogenizing apparatus and at a homogenization speed of between 2500 rpm and 10,000 rpm. In another even more preferred embodiment, the method is characterized in that said homogenization is carried out for a time between 2 minutes and 30 minutes.

In a preferred embodiment of the method of the present invention, the method is characterized in that the step of fixing said microcapsules to said fabric comprises the steps of: obtaining an aqueous suspension containing said microcapsules; adding a binding agent to said aqueous suspension; and immersing said fabric into said suspension containing said binding agent. In a more preferred embodiment, the method is characterized in that said binding agent is selected from the group consisting of: INDOSOL E-50, TUBIPRINT BINDER MD, TUBIPRINT BINDER CH 1239 FF, ECCOBOND™ OLF, ECCO™ Low Crock NCN 313-524, ECCO™ Low Crock NP-5, ECCO™ Resin Binder 4501, Lenospin™ SDS-85, Orco Air Dry Binder 6398™, Nylofixan® HF liq CG5, Nylofixan® HF liq c CG5, Optifix CLC liq, Optifix EC liq, and Optifix RSL liq Suparex O.IN p CG9, as well as combinations thereof.

In another more preferred embodiment of the invention, the method is characterized in that said aqueous suspension has a microcapsule concentration of between 8×10⁸ and 3.5×10⁹ microcapsules per ml. In an additional more preferred embodiment, the method is characterized in that said aqueous suspension has a binding agent concentration of between 5% and 15% v/v. In another even more preferred embodiment, the method is characterized in that said aqueous suspension is maintained at a temperature between 30°C and 50°C.

### DETAILED DESCRIPTION OF THE INVENTION

In a first object of the invention, the present invention provides a material for the release of vitamin D directly to the skin that comprises, essentially, a fabric and microcapsules containing vitamin D dispersed in said fabric and fixed thereto, wherein each of said microcapsules is formed by an amphipathic polymeric matrix encapsulating said vitamin D.

With respect to said fabric, it can be made of natural, artificial, or synthetic fibers, as well as combinations thereof, without limiting the scope of the present invention. For example, and without limiting the scope of the present invention, said fabric may be selected from the group consisting of wool, silk, linen, cotton, hemp, nylon, polyester, polyethylene, polypropylene, elastomer, as well as combinations thereof.

Optionally, without limiting the scope of the present invention, said fabric may incorporate metallic fibers or microfibers. In the state of the art, it is known that some metallic materials have antimicrobial properties, for example, silver or copper, reason why fibers made of said materials have been incorporated into fabrics to provide them with said properties. In a preferred embodiment, without limiting the scope of the present invention, said fabric comprises copper microfibers.

On the other hand, with respect to said microcapsules containing vitamin D, it should be understood, in the context of the present invention, that a microcapsule is generally formed as a particle having a core that is encapsulated by a coating, and wherein the diameter of said particle is on the microscale, for example, and without limiting the scope of the present invention, in the range between 0.1 microns and 500 microns. On the other hand, the specific shape of said microcapsule, as well as other geometric properties such as the aspect ratio, do not limit the scope of the present invention.

Hereinafter, when referring to a microcapsule to describe the structural aspects thereof, it should be understood that it refers to one or more microcapsules sharing said structural aspects.

In the case of the present invention, said microcapsule is a vitamin D microcapsule encapsulated in an amphipathic polymeric matrix. The use of an amphipathic polymer as part of said amphipathic polymeric matrix allows, on the one hand, said amphipathic polymer to form a shell that surrounds said vitamin D while allowing a slow release of vitamin D through said amphipathic polymeric matrix.

The nature of said amphipathic polymeric matrix does not limit the scope of the present invention and may comprise molecules selected from the group consisting of phospholipids, cholesterols, glycolipids, fatty acids, saponins, caseins, as well as combinations thereof. In a preferred embodiment, without limiting the scope of the present invention, said polymeric matrix comprises casein. In a more preferred embodiment, without limiting the scope of the present invention, said amphipathic polymeric matrix comprises sodium caseinate. However, other options are possible, such as acacia gum and Capsul®, as well as combinations of these molecules with the previously listed options.

Said polymeric matrix may comprise, additionally and without limiting the scope of the present invention, other types of molecules such as, for example, surfactants. In a preferred embodiment, wherein said polymeric matrix comprises an amphipathic molecule that is selected from the group consisting of sodium caseinate, acacia gum, Capsul®, as well as combinations thereof, said polymeric matrix may comprise an emulsifying agent having a hydrophilic-lipophilic balance between 10 and 20. In a preferred embodiment, without limiting the scope of the present invention, said emulsifying agent is polysorbate 20.

Regarding the core of said microcapsule, it comprises vitamin D. However, other compounds may be included inside said microcapsule without limiting the scope of the present invention. For example, said microcapsule may additionally contain in its core a compound selected from the group consisting of liquid petroleum jelly, vegetable oil, triolein, as well as combinations thereof. Additionally, without limiting the scope of the present invention, said microcapsule may comprise compounds other than vitamin D. For example, and without limiting the scope of the present invention, said microcapsule may comprise carotene. This last preferred embodiment has the advantage that it allows to visually discriminate the presence and fixation of microcapsules in the fabric, as well as the subsequent release of vitamin D therefrom.

On the other hand, the size of said microcapsule does not limit the scope of the present invention as long as it is within the previously defined range. In a preferred embodiment, said microcapsule has a diameter size between 0.1 microns and 20 microns, more preferably between 0.5 microns and 4 microns, and even more preferably between 0.7 microns and 3.2 microns.

The content of vitamin D in the microcapsule does not limit the scope of the present invention. A person having ordinary skill in the art will observe that the content of vitamin D in the microcapsule will depend, among other aspects, on the method whereby said microcapsules are obtained or manufactured. Said vitamin D content may be, for example and without limiting the scope of the present invention, between 2% and 25% by weight of the total weight of the microcapsule.

Regarding the content of microcapsules that contains the material that is the object of the present invention, the same does not limit the scope of the present invention and may have any value that allows adequate release of vitamin D through the skin. In a preferred embodiment, the concentration of said microcapsules containing vitamin D in said fabric is between 0.5 and 2 mg of vitamin D per square meter of fabric.

The present invention further provides a method for obtaining a material for the release of vitamin D through the skin which comprises, essentially, the steps of: obtaining microcapsules containing vitamin D encapsulated in an amphipathic polymeric matrix; and fixing said microcapsules containing vitamin D to a fabric.

The manner in which said microcapsules containing vitamin D are obtained does not limit the scope of the present invention and any method known in the state of the art may be used, as well as obtaining said microcapsules from a commercial supplier, without limiting the scope of the present invention.

In a preferred embodiment, said microcapsules containing vitamin D encapsulated in an amphipathic polymeric matrix are obtained by the emulsion-separation method. Said method comprises the steps of: obtaining an oil phase containing vitamin D; obtaining an aqueous phase containing an aqueous solution of a precursor of said amphipathic polymeric matrix; adding said oil phase to said aqueous phase while homogenizing said mixture; and recovering said microcapsules containing vitamin D.

The volume ratio between said oil phase and said aqueous phase does not limit the scope of the present invention and may be, for example and without limiting the scope of the present invention, between 1:10 and 1:2. In a more preferred embodiment, said volume ratio is between 1:6 and 1:3, and more preferably is 1:4.

On the other hand, said oil phase may comprise vitamin D, either alone or in a solution, without limiting the scope of the present invention. In a preferred embodiment, without limiting the scope of the present invention, said oil phase comprises an oil solution of vitamin D. In a more preferred embodiment, without limiting the scope of the present invention, said oil solution of vitamin D may comprise any oil solvent. For example, said oil solvent can be selected from the group consisting of liquid petroleum jelly, vegetable oil, triolein, as well as combinations thereof. Additionally, the concentration of vitamin D in said oil phase does not limit the scope of the present invention and will depend, among other aspects, on the desired content of vitamin D in the microcapsules. In a preferred embodiment, without limiting the scope of the present invention, the concentration of vitamin D in said oil phase is between 100 ppm and 1000 ppm. Additionally, said oil phase may comprise other compounds without limiting the scope of the present invention. For example, said oil phase may comprise a carotene, which is used as a colorant and allows to visually verify the fixation of microcapsules to the fabric that is part of the material that is the object of the present invention.

Said aqueous phase, for its part, comprises an aqueous solution of a precursor of said amphipathic polymeric matrix. In this sense, a precursor of said polymeric matrix should be understood as a compound or mixture of compounds that, once the microcapsule is formed, will form said polymeric matrix. The nature of said precursor of said polymeric matrix does not limit the scope of the present invention and may be chosen from the group consisting of phospholipids, cholesterols, glycolipids, fatty acids, saponins, caseins, as well as combinations thereof. In a preferred embodiment, without limiting the scope of the present invention, said precursor of said polymeric matrix comprises a casein. In another more preferred embodiment, without limiting the scope of the present invention, said precursor of said polymeric matrix comprises an amphipathic molecule that is selected from the group consisting of sodium caseinate, acacia gum, and Capsul®, as well as combinations thereof. In an even more preferred embodiment, said amphipathic molecule is sodium caseinate.

The concentration of said precursor of said polymeric matrix in said aqueous phase does not limit the scope of the present invention and will depend, for example, on the conditions in which said aqueous phase is obtained, as well as the solubility limit of said precursor of said polymeric matrix. Said concentration may be, for example and without limiting the scope of the present invention, between 2% and 50% w/w, more preferably between 5% and 20% w/w, and even more preferably between 7% and 15% w/w. In the preferred embodiment in which said precursor of said polymeric matrix is sodium caseinate, the concentration of sodium caseinate in said aqueous phase can be between 7% and 15% w/w.

Optionally, without limiting the scope of the present invention, said aqueous phase may comprise one or more surfactants. If present, the concentration of said surfactant in said aqueous phase does not limit the scope of the present invention and may be, for example and without limiting the scope of the present invention, between 1% and 20% w/w, more preferably between 7% and 15% w/w. In a preferred embodiment, said aqueous phase additionally comprises an emulsifying agent having a hydrophilic-lipophilic balance between 10 and 20. In an even more preferred embodiment, said emulsifying agent is polysorbate 20. The concentration of polysorbate 20 in said aqueous phase can be between 7% and 15% w/w without limiting the scope of protection.

The manner in which said aqueous phase is obtained does not limit the scope of the present invention. For example, said aqueous phase may be obtained by homogenizing said precursor of said polymeric matrix and water by means of a dispersing homogenizing apparatus, commonly known as Ultra-Turrax. In this last preferred embodiment, the speed of homogenization does not limit the scope of the present invention and may be, for example and without limiting the scope of the present invention, between 1000 rpm and 15,000 rpm, more preferably between 2500 rpm and 10,000 rpm. On the other hand, the homogenization time also does not limit the scope of the present invention and may be, for example and without limiting the scope of the present invention, between 1 minute and 100 minutes, more preferably between 2 minutes and 30 minutes. Additionally, the temperature at which said homogenization is carried out does not limit the scope of the present invention and can be carried out, for example and without limiting the scope of the present invention, at a temperature between 20°C and 90°C, more preferably higher than 40°C, and even more preferably at a temperature between 60°C and 80°C. On the other hand, the pH at which said homogenization is carried out does not limit the scope of the present invention and can be controlled by any method known in the state of the art. In a preferred embodiment, said homogenization is carried out at a pH between 4 and 10, more preferably between 5 and 8, and even more preferably between 6.5 and 7.5.

As previously mentioned, said microcapsules containing vitamin D can be obtained by a method comprising the step of adding said oil phase to said aqueous phase while homogenizing said mixture. In this sense, said homogenization may be carried out by any technique known in the state of the art without limiting the scope of the present invention. In a preferred embodiment, said homogenization is carried out by means of a dispersing homogenizing apparatus. In this last preferred embodiment, the speed of homogenization does not limit the scope of the present invention and may be, for example and without limiting the scope of the present invention, between 1000 rpm and 15,000 rpm, more preferably between 2500 rpm and 10,000 rpm. On the other hand, the homogenization time also does not limit the scope of the present invention and may be, for example and without limiting the scope of the present invention, between 1 minute and 100 minutes, more preferably between 2 minutes and 30 minutes. Additionally, the temperature at which said homogenization is carried out does not limit the scope of the present invention and may be carried out, for example and without limiting the scope of the present invention, at a temperature between 20°C and 90°C, more preferably higher than 40°C, and even more preferably at a temperature between 60°C and 80°C. On the other hand, the pH at which said homogenization is carried out does not limit the scope of the present invention and may be controlled by any method known in the state of the art. In a preferred embodiment, said homogenization is carried out at a pH between 4 and 10, more preferably between 5 and 8, and even more preferably between 6.5 and 7.5.

On the other hand, to obtain the material that is the object of the present invention, the method that is the object of the present invention comprises the step of fixing said microcapsules to said fabric. In a preferred embodiment, without limiting the scope of the present invention, said step of fixing said microcapsules to said fabric comprises the steps of: obtaining an aqueous suspension containing said microcapsules; adding a binding agent to said aqueous suspension; and immersing said fabric into said suspension containing said binding agent.

The function of said binding agent, without limiting the scope of the present invention, is to provide a better fixation of said microcapsules of said fabric. Surprisingly, in the context of the present invention, it has been found that any binding agent commonly used in the textile industry to add a pigment to a fabric may be used to fix said microcapsules to said fabric. In a preferred embodiment, without limiting the scope of the present invention, said binding agent is selected from the group consisting of: INDOSOL E-50, TUBIPRINT BINDER MD, TUBIPRINT BINDER CH 1239 FF, ECCOBOND™ OLF, ECCO™ Low Crock NCN 313-524, ECCO™ Low Crock NP-5, ECCO™ Resin Binder 4501, Lenospin™ SDS-85, as well as combinations thereof. Additionally, the binding agent concentration of in said aqueous phase does not limit the scope of the present invention and may be, for example and without limiting the scope of the present invention, between 2% and 30%, more preferably between 5% and 15%.

On the other hand, the content of microcapsules in said aqueous suspension does not limit the scope of the present invention and will depend on the number of microcapsules that is intended to fix to the fabric, as well as the desired concentration of microcapsules in the material that is object of the present invention. In a preferred embodiment, without limiting the scope of the present invention, the concentration of microcapsules in said aqueous phase is between 8×10⁸ and 3.5×10⁹ microcapsules per ml.

The temperature at which said method to fix said microcapsules to said fabric is carried out does not limit the scope of the present invention. In a preferred embodiment, said temperature is between 20°C and 60°C, more preferably between 30°C and 50°C, and even more preferably at 40°C. This temperature range also coincides with the temperature range commonly used in the textile industry to fix pigments to fabrics using a binding agent. Advantageously, the method for fixing microcapsules containing vitamin D to a fabric may be implemented in the textile industry without requiring major modifications in the methods commonly used in said industry.

According to the previously detailed description, it is possible to obtain a material for the release of vitamin D through the skin, as well as a method to obtain the same.

Hereinafter, embodiments of the present invention will be described. It should be understood that such examples are provided solely for the purpose of illustrating and providing a better understanding of the present invention, but should in no way be construed to limit the scope of protection.

Additionally, technical characteristics present in different examples may be combined with each other, or with previously detailed technical characteristics, in any way that is obvious to a person having ordinary skill in the art, without limiting the scope of the present invention.

### Example 1: Obtaining aqueous phase and oil phase.

Microcapsules were obtained by the emulsion-separation method, using to this end an aqueous phase and an oil phase.

The aqueous phase consisted of a solution of 10% by weight sodium caseinate and 10% by weight polysorbate 20 in distilled water. To obtain it, water and a magnetic stirrer were added in a beaker, and the water was heated to 70°C. Subsequently, polysorbate 20 was added, and then sodium caseinate was added at a very slow rate. Agitation of the mixture was maintained at all times while both polysorbate 20 and sodium caseinate were added.

The oil phase, for its part, consisted entirely of vitamin D.

The volume ratio between said oil phase and said aqueous phase was 1:5.

### Example 2: Obtaining vitamin D microcapsules.

After preparing the aqueous phase, the entire oil phase was added to it. Subsequently, the mixture was stirred in a homogenizer (Ultra-Turrax) at three different homogenization speeds and for five different homogenization times, thus obtaining 15 speed-time combinations to obtain microcapsules.

The homogenization speeds used were 2500 rpm, 5000 rpm, and 10,000 rpm, while the homogenization times used were 2 minutes, 5 minutes, 10 minutes, 20 minutes, and 30 minutes.

The results of the size of the microcapsules obtained are shown in Table 1.

**Table 1: Average size of microcapsules as a function of speed and homogenization time**

| **Speed (rpm)** | **Time (min)** | **Mean Diameter (µm)** | **Standard Deviation (µm)** | **No. of microcapsules measured** |
|---|---|---|---|---|
| 2500 | 2 | 2.60 | 1.05 | 166 |
| | 5 | 1.67 | 0.37 | 244 |
| | 10 | 1.59 | 0.86 | 182 |
| | 20 | 1.30 | 0.41 | 348 |
| | 30 | 1.01 | 0.24 | 243 |
| 5000 | 2 | 2.02 | 0.81 | 239 |
| | 5 | 0.95 | 0.33 | 249 |
| | 10 | 1.30 | 0.40 | 280 |
| | 20 | 0.72 | 0.22 | 253 |
| | 30 | 1.78 | 0.46 | 248 |
| 10,000 | 2 | 1.20 | 0.34 | 244 |
| | 5 | 2.17 | 0.85 | 252 |
| | 10 | 1.50 | 0.60 | 267 |
| | 20 | 1.18 | 0.31 | 250 |
| | 30 | 1.69 | 0.53 | 244 |

### Example 3: Fixing microcapsules to the fabric

To carry out the process of fixing the microcapsules to the fabric, a 1:500 suspension of microcapsules in distilled water was obtained. Subsequently, a part of said suspension was added to a beaker and a volume of Indosol E-50 binding agent was added corresponding to a concentration of 5%, 10%, or 15% by volume, out of a total of 50 ml of solution. This solution was heated on a magnetic stirrer to 47°C, and then a square of *polycotton* cloth of an area of 1 cm² was added to it, maintaining stirring throughout the process. Additionally, fixation times of between 0 and 120 minutes were tested.

It was found that in all three concentrations of binding agent used, the microcapsules were successfully fixed to the fabric. On the other hand, it was found that at a fixation time of 40 minutes, under the concentration and temperature conditions previously described, the fixation of microcapsules to the fabric reached a maximum, which was maintained for longer fixation times.

## Claims

1. A material for the release of vitamin D through the skin, **CHARACTERIZED in that** it comprises:
- a fabric; and
- microcapsules containing vitamin D dispersed in said fabric and fixed thereto, wherein each of said microcapsules is formed by an amphipathic polymeric matrix encapsulating said vitamin D.

2. The material of claim 1, **CHARACTERIZED in that** said fabric is selected from the group consisting of cotton fabric, polyester fiber fabric, as well as combinations thereof.

3. The material of claim 1, **CHARACTERIZED in that** said fabric comprises copper microfibers.

4. The material of claim 1, **CHARACTERIZED in that** said polymeric matrix comprises an amphipathic polymer that is selected from the group consisting of sodium caseinate, acacia gum, and Capsul®, as well as combinations thereof.

5. The material of claim 4, **CHARACTERIZED in that** said amphipathic polymer is sodium caseinate.

6. The material of claim 4, **CHARACTERIZED in that** said polymeric matrix comprises an emulsifying agent with a hydrophilic-lipophilic balance between 10 and 20.

7. The material of claim 6, **CHARACTERIZED in that** said emulsifying agent is polysorbate 20.

8. The material of claim 1, **CHARACTERIZED in that** said microcapsules have a diameter size between 0.7 microns and 3.2 microns.

9. The material of claim 1, **CHARACTERIZED in that** the concentration of said microcapsules in said fabric is between 0.5 and 2 mg of vitamin D per square meter of fabric.

10. A method for obtaining a material for the release of vitamin D through the skin, **CHARACTERIZED in that** it comprises the steps of:
- obtaining microcapsules containing vitamin D encapsulated in an amphipathic polymeric matrix; and
- fixing said microcapsules containing vitamin D to a fabric.

11. The method of claim 10, **CHARACTERIZED in that** the step of obtaining said microcapsules containing vitamin D encapsulated in an amphipathic polymeric matrix comprises the steps of:
- obtaining an oil phase containing vitamin D;
- obtaining an aqueous phase containing an aqueous solution of a precursor of said amphipathic polymeric matrix;
- adding said oil phase to said aqueous phase while homogenizing the mixture; and
- recovering said microcapsules containing vitamin D.

12. The method of claim 11, **CHARACTERIZED in that** the volume ratio between said oil phase and said aqueous phase is between 1:10 and 1:2.

13. The method of claim 11, **CHARACTERIZED in that** said oil phase comprises an oil solution of said vitamin D.

14. The method of claim 13, **CHARACTERIZED in that** said oil solution of vitamin D comprises a solvent that is selected from the group consisting of liquid petroleum jelly, vegetable oil, triolein, as well as combinations thereof.

15. The method of claim 11, **CHARACTERIZED in that** said oil phase contains a concentration of vitamin D between 100 ppm and 1000 ppm.

16. The method of claim 11, **CHARACTERIZED in that** said precursor of said polymeric matrix comprises an amphipathic polymer that is selected from the group consisting of sodium caseinate, acacia gum, and Capsul®, as well as combinations thereof.

17. The method of claim 16, **CHARACTERIZED in that** said amphipathic polymer is sodium caseinate.

18. The method of claim 17, **CHARACTERIZED in that** the concentration of sodium caseinate in said aqueous phase is between 7% and 15% w/w.

19. The method of claim 11, **CHARACTERIZED in that** said aqueous phase additionally contains an emulsifying agent having a hydrophilic-lipophilic balance between 10 and 20.

20. The method of claim 19, **CHARACTERIZED in that** said emulsifying agent is polysorbate 20.

21. The method of claim 20, **CHARACTERIZED in that** the concentration of polysorbate 20 in said aqueous phase is between 7% and 15% w/w.

22. The method of claim 11, **CHARACTERIZED in that** the step of obtaining said aqueous phase comprises homogenizing the precursor of said polymeric matrix and water by means of a dispersing homogenizing apparatus.

23. The method of claim 22, **CHARACTERIZED in that** said homogenization is carried out at a temperature between 60°C and 80°C.

24. The method of claim 11, **CHARACTERIZED in that** said homogenization is carried out by means of a dispersing homogenizing apparatus.

25. The method of claim 24, **CHARACTERIZED in that** said homogenization is carried out a homogenization speed of between 2500 rpm and 10,000 rpm.

26. The method of claim 24, **CHARACTERIZED in that** said homogenization is carried out for a time between 2 minutes and 30 minutes.

27. The method of claim 10, **CHARACTERIZED in that** the step of fixing said microcapsules to said fabric comprises the steps of:
- obtaining an aqueous suspension containing said microcapsules;
- adding a binding agent to said aqueous suspension; and
- immersing said fabric into said suspension containing said binding agent.

28. The method of claim 27, **CHARACTERIZED in that** said binding agent is selected from the group consisting of INDOSOL E-50, TUBIPRINT BINDER MD, TUBIPRINT BINDER CH 1239 FF, ECCOBOND™ OLF, ECCO™ Low Crock NCN 313-524, ECCO™ Low Crock NP-5, ECCO™ Resin Binder 4501, and Lenospin™ SDS-85, as well as combinations thereof.

29. The method of claim 27, **CHARACTERIZED in that** said aqueous suspension has a microcapsule concentration of between 8×10⁸ and 3.5×10⁹ microcapsules per ml.

30. The method of claim 27, **CHARACTERIZED in that** said aqueous suspension has a binding agent concentration of between 5% and 15% v/v.

31. The method of claim 27, **CHARACTERIZED in that** said aqueous suspension is maintained at a temperature between 30°C and 50°C.
